(19) Europäisches Patentamt
European Patent Office
Office européen des brevets

(11) **EP 3 923 901 B1**

(12) **FASCICULE DE BREVET EUROPEEN**

(45) Date de publication et mention de la délivrance du brevet:
**13.12.2023 Bulletin 2023/50**

(21) Numéro de dépôt: **20702851.5**

(22) Date de dépôt: **10.02.2020**

(51) Classification Internationale des Brevets (IPC):
**A61K 8/44** *(2006.01)*    **A61Q 19/02** *(2006.01)*
**A61K 8/64** *(2006.01)*

(52) Classification Coopérative des Brevets (CPC):
**A61K 8/447; A61K 8/64; A61Q 19/02;**
A61K 2800/5922; A61K 2800/92; A61Q 19/08

(86) Numéro de dépôt international:
**PCT/EP2020/053311**

(87) Numéro de publication internationale:
**WO 2020/165084 (20.08.2020 Gazette 2020/34)**

(54) **UTILISATION NON-THÉRAPEUTIQUE PAR VOIE ORALE D'UNE COMPOSITION POUR BLANCHIR ET/OU ÉCLAIRCIR LA PEAU COMPRENANT DE LA CYSTINE ET DU GLUTATHION DANS UN RAPPORT CYSTINE/GLUTATHION ALLANT DE 1,5 À 4**

NICHT-THERAPEUTISCHE ORALE VERWENDUNG EINER ZUSAMMENSETZUNG ZUM BLEICHEN UND/ODER AUFHELLEN DER HAUT MIT CYSTIN UND GLUTATHION IN EINEM CYSTEIN-GLUTATHION-VERHÄLTNIS IM BEREICH VON 1,5 BIS 4

NON-THERAPEUTIC ORAL USE OF A COMPOSITION FOR WHITENING AND/OR LIGHTENING THE SKIN COMPRISING CYSTINE AND GLUTATHIONE IN A CYSTINE-GLUTATHIONE RATIO RANGING FROM 1.5 TO 4

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.02.2019 FR 1901355**

(43) Date de publication de la demande:
**22.12.2021 Bulletin 2021/51**

(73) Titulaire: **Bretagne Chimie Fine**
**56140 Pleucadeuc (FR)**

(72) Inventeurs:
• **DUPERRAY, Joel**
**56250 TREFFLEAN (FR)**

• **SERGHERAERT, Renaud**
**56870 BADEN (FR)**

(74) Mandataire: **Oak & Fox**
**94, rue La Fayette / Esc. D**
**75010 Paris (FR)**

(56) Documents cités:
**WO-A1-2016/117762    WO-A2-2007/116428**
**KR-A- 20120 025 175**

Remarques:
Le dossier contient des informations techniques présentées postérieurement au dépôt de la demande et ne figurant pas dans le présent fascicule.

**Description**

La présente invention se rapporte au domaine des compositions cosmétiques administrées par voie orale présentant une action blanchissante et/ou éclaircissante et/ou dépigmentante de la peau.

On note depuis plusieurs années une nette recrudescence de la demande des consommateurs pour des compositions blanchissantes et/ou éclaircissantes de la peau.

En Asie, le recours à ce type de produits est très courant car la recherche d'une peau claire répond à un critère esthétique ancien et toujours très recherché. Plus globalement, à différentes périodes de leur vie, certaines personnes voient apparaître sur leur peau notamment sur le visage et les mains, des taches plus foncées qui génèrent des zones d'aspect non-homogène.

Ces taches sont généralement dues à une concentration importante de mélanine dans les kératinocytes situés à la surface de la peau. En effet, la mélanine biologique est le pigment responsable de la coloration de la peau, elle est constituée d'eumélanine et de phéomélanine formées dans les mélanocytes de la couche basale de la peau.

L'eumélanine est le type le plus répandu de mélanine tégumentaire, elle est de couleur brune à noire. Sa présence en quantité importante donne aux téguments, notamment à la peau, une couleur brun foncé ou noir, son pouvoir de protection contre les dommages causés à l'épiderme par les rayons UV est total. L'eumélanine est formée d'un mélange de macromolécules d'acide 5,6 dihydroxy-indole-2-carboxylique (DHICA) et de 5,6-dihydroxyindole (DHI).

Le mécanisme de formation de l'eumélanine, est particulièrement complexe et fait intervenir schématiquement les principales étapes suivantes :

**Tyrosine→DOPA→Dopaquinone →leucodopachrome→dopachrome → acide 5,6 dihydroxyindole-2-carboxylique et/ou 5,6 dihydroxyindole**

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase.

La phéomélanine est de couleur plus claire que l'eumélanine, sa couleur est jaune à rouge. La phéomélanine est également formée d'un mélange de macromolécules synthétisées dans les mélanocytes à partir de la tyrosine. En présence de cystéine intracellulaire, une certaine quantité de tyrosine est transformée en cystéinyldopa, puis un en polymère dont le monomère principal contient du benzothiazole et de la dihydroisoquinoline :

**Tyrosine→DOPA→Dopaquinone** L-cystéine intracellulaire **→cystéinyldopa→ benzothiazole + dihydoisoquinoline**

Il existe déjà de nombreux produits connus pour leur activité blanchissante et/ou dépigmentante comme par exemple l'hydroquinone, l'acide kojique, l'arbutine, le glutathion, certains flavonoides.

Ainsi une substance est reconnue comme blanchissante ou dépigmentante si elle agit directement sur la vitalité des mélanocytes épidermiques où se déroule la mélanogénèse, et/ou si elle interfère avec une des étapes de la biosynthèse de la mélanine soit en inhibant une des enzymes impliquées dans la mélanogénèse, soit en s'intercalant comme analogue structural d'un des composés chimiques de la chaîne de synthèse de la mélanine, chaîne qui peut alors être bloquée et ainsi assurer la dépigmentation, et donc l'éclaircissement et/ou le blanchissement de la peau.

Certains produits blanchissants/dépigmentants sont connus pour leur action d'inhibition de l'enzyme tyrosinase qui a pour effet de conduire à une diminution de la synthèse de la phéomélanine et de l'eumélanine. Cette diminution de la quantité d'eumélanine a certes un effet blanchissant/dépigmentant mais contribue aussi à diminuer le pouvoir naturel de protection de la peau contre les dommages causés par les rayons UV.

Le glutathion est connu pour son haut pouvoir anti-oxydant intracellulaire, il constitue la première ligne de défense contre les radicaux libres. Il est également connu dans l'industrie cosmétique pour ses effets blanchissants et/ou éclaircissants de la peau par voie topique. Cependant lorsqu'il est utilisé par voie orale, sa biodisponibilité est controversée au motif, qu'en l'absence d'un véhicule spécifique au niveau de la membrane cellulaire, il est susceptible d'être dégradé par les peptidases du tube digestif.

La demande de brevet WO2016/117762 A1 divulgue l'utilisation d'une composition, notamment destinée à la voie orale, pour blanchir la peau comprenant un extrait de groseille à maquereau et du glutathion.

En outre, la demande de brevet KR 2012 0025175 A décrit une composition pour améliorer le teint de la peau ou pour blanchir la peau comprenant un extrait de mandarine satsuma, de cystine et de vitamine C.

Il demeure par conséquent toujours un besoin de nouvelles compositions destinées à la voie orale présentant de bonnes propriétés dépigmentantes et/ou blanchissantes pour éclaircir l'ensemble du visage et/ou du corps ou éclaircir et rendre plus homogène des zones plus précises du visage et/ou du corps. Ces compositions doivent en outre être stables, non irritantes, non toxiques, non allergisantes pour la peau.

De manière surprenante et avantageuse les auteurs de la présente invention sont parvenus à surmonter les problèmes de l'art antérieur et ont montré que l'utilisation, par voie orale, d'une composition comprenant au moins de la cystine et du glutathion en un rapport particulier permet d'obtenir des propriétés très satisfaisantes en termes de blanchiment, éclaircissement, dépigmentation de la peau et en particulier pour réduire la taille des taches brunes pigmentaires.

D'autres aspects, avantages, propriétés de la présente invention sont présentés dans la description et les exemples qui suivent.

La présente invention a pour objet l'utilisation non-thérapeutique, cosmétique, par voie orale, d'une composition à titre d'actif pour blanchir et/ou éclaircir la peau et/ou dépigmenter la peau, en particulier pour réduire la taille des taches brunes, ladite composition comprenant au moins de la cystine et du glutathion en un rapport cystine/glutathion allant de 1,5 à 4, de préférence 1,5 à 3, et de manière préférée de 1,5 à 2,5 et avantageusement de 1,8 à 2,2.

Elle vise encore un procédé non-thérapeutique, cosmétique de blanchiment et/ou d'éclaircissement et/ou de dépigmentation de la peau comprenant une étape d'administration à un individu par voie orale d'une composition comprenant au moins de la cystine et du glutathion en un rapport cystine/glutathion allant de 1,5 à 4, de préférence 1,5 à 3, et de manière préférée de 1,5 à 2,5 et avantageusement de 1,8 à 2,2.

Un avantage de la composition selon la présente invention est qu'elle possède des propriétés de blanchiment et/ou éclaircissement et/ou dépigmentation de la peau et notamment de réduction de la taille des taches brunes pigmentaires très satisfaisantes.

Dans le présent texte, les expressions « blanchiment », «éclaircissement » et « dépigmentation » utilisées pour définir les propriétés des compositions selon l'invention ont la même signification, de même que les expressions « blanchir », « éclaircir » et « dépigmenter ». En particulier ces expressions ne visent pas un processus mettant en oeuvre l'inhibition d'une enzyme conduisant à empêcher toute formation de mélanines. En effet la composition selon l'invention présente également l'avantage de garantir en partie le pouvoir naturel de protection de la peau vis-à-vis des dommages causés par les rayons UV. Par « peau », on entend la peau du visage et/ou du corps, de préférence du visage.

Sans vouloir être lié à une quelconque théorie, il semble que la composition selon l'invention présentant un rapport cystine/glutathion précis permet d'augmenter de manière significative la concentration en cystéine intracellulaire et de maintenir un taux de cystéine/glutathion intracellulaire, elle permet également d'orienter la voie de synthèse de la mélanine vers une augmentation de la production de phéomélanine et une diminution de la production d'eumélanine. En conséquence, en comparaison avec les substances de l'art antérieur qui ont pour effet d'inhiber l'action de l'enzyme tyrosinase et donc d'inhiber la formation des eumélanine et phéomélanine, la composition selon l'invention permet toujours la synthèse de phéomélanine. Or, la phéomélanine possède un pouvoir d'absorption des UV certain même s'il est inférieur à celui de l'eumélanine.

La composition selon l'invention présente également les avantages de conférer une peau plus lumineuse, plus rayonnante et homogène, en particulier une carnation plus nette, un teint d'aspect plus frais, plus reposé, c'est-à-dire un effet « bonne mine ».

Elle favorise aussi le renouvellement des kératinocytes qui constituent les cellules de l'épiderme, couche superficielle de la peau.

La composition selon l'invention permet d'atténuer voire de traiter un teint de peau brouillé, terne, hétérogène et plus particulièrement de traiter les dyschromies et de réduire la taille des taches brunes.

La composition selon l'invention est administrée par la voie orale permettant une action blanchissante/ éclaircissante efficace et durable sur la totalité de la peau.

Description détaillée de l'invention

La composition utilisée selon l'invention comprend de la cystine qui est un acide aminé composé de deux monomères de cystéine liés par un pont disulfure.

De préférence, la cystine utilisée est une cystine très pure dans laquelle les impuretés principales sont à l'états de traces, en particulier la cystine utilisé comprend moins de 5 ppm de métaux lourds, avantageusement elle comprend moins de 5 ppm de métaux lourds, moins de 100 ppm de sulfates et moins de 0,004% d'ammonium. Le dosage des métaux lourds est effectué par colorimétrie en présence de thioacétamide (Ph. Eur. 9eme Ed. 2.4.8, essai limite D), le dosage des sulfates est effectué en présence de sels de baryum, en milieu acide (Ph. Eur. 9eme Ed 2.4.13), le dosage de l'ammonium est effectué par chromatographie par échanges d'ions avec dérivatisation post-colonne par la ninhydrine (Ph. Eur. 9eme Ed 2.2.56). De manière préférée la cystine utilisée dans la présente invention est la cystine commercialisée sous la dénomination Traced L-Cystine par la société BCF Life Sciences.

La cystine est utilisée dans la composition selon l'invention sous forme d'un acide aminé libre, avantageusement la quantité de cystine dans ladite composition va de 30 à 60%, de préférence de 35 à 45 % poids par rapport au poids total de ladite composition.

La composition utilisée selon l'invention comprend aussi du glutathion. Le glutathion est un pseudo-tripeptide formé par la condensation d'acide glutamique, de cystéine et de glycine. Le glutathion est utilisé dans la composition selon l'invention sous forme libre. En particulier, le glutathion utilisé dans la présente invention est du glutathion en tant que tel, sous forme peptidique, il n'est pas sous forme d'une levure.

En effet, dans certaines applications dans lesquelles l'utilisation du glutathion n'est pas autorisée, des levures sont utilisées à la place du glutathion. Les levures comprennent généralement du glutathion en une quantité qui n'est cependant pas toujours déterminable. En outre l'activité/l'efficacité d'une quantité donnée de levure ne correspond pas toujours à l'activité/l'efficacité de la même quantité de glutathion sous forme libre. Par conséquent, lorsque des levures sont utilisées, il peut être difficile d'établir le rapport cystine/glutathion.

Avantageusement le glutathion utilisé dans la composition selon la présente invention est sous forme réduite dite forme « GSH ».

De préférence, la quantité de glutathion dans la composition utilisée selon l'invention va de 10 à 30 %, de préférence de 15 à 25 % poids par rapport au poids total de ladite composition.

Avantageusement la composition utilisée selon l'invention comprend de la glutamine. La glutamine est un acide aminé utilisé dans la composition selon l'invention sous forme libre.

De préférence, la glutamine est utilisée dans la composition selon l'invention dans un rapport cystine/glutamine allant de 3,5 à 5,0, de manière préférée allant de 4,0 à 4,5.

De préférence, la quantité de glutamine dans la composition utilisée selon l'invention va de 3 à 20 %, de préférence de 5 à 15 % poids par rapport au poids total de ladite composition.

Avantageusement la composition utilisée selon l'invention comprend de la glycine. La glycine est un acide aminé utilisé dans la composition selon l'invention sous forme libre.

De préférence, la glycine est utilisée dans la composition selon l'invention dans un rapport cystine/glycine allant de 7 à 9,5, de manière préférée allant de 8,0 à 8,5.

De préférence, la quantité de glycine dans la composition utilisée selon l'invention va de 1 à 10 %, de préférence de 2 à 8 % poids par rapport au poids total de ladite composition.

Avantageusement la composition utilisée selon l'invention comprend de l'ascorbate de calcium, encore appelé « vitamine C ».

De préférence, l'ascorbate de calcium est utilisé dans la composition selon l'invention dans un rapport cystine/ ascorbate de calcium allant de 3,5 à 5,5, de manière préférée allant de 4,0 à 5,0.

De préférence, la quantité d'ascorbate de calcium dans la composition utilisée selon l'invention va de 3 à 20 %, de préférence de 5 à 15 % poids par rapport au poids total de ladite composition.

La composition utilisée selon l'invention comprend au moins un composant choisi dans le groupe formé par le zinc ou l'un de ses sels, le cuivre ou l'un de ses sels, le sélénium ou l'un de ses sels, les vitamines B, notamment B3, B5, B6 et/ou B8, la vitamine E, les extraits de jus de melon et les extraits de prêle, ainsi que les mélanges de ces composants.

De manière générale, au sens du présent texte, on entend par « sel » tout sel qui présente une innocuité adéquate.

La quantité de vitamine B3 de la composition utilisée selon la présente invention va de 0,5 à 10 %, de préférence de 1 à 5 % en poids par rapport au poids total de ladite composition.

La vitamine B3 (ou vitamine PP) peut être présente dans le complément alimentaire sous la forme d'un ou plusieurs composés tels que le nicotinamide (ou niacinamide), l'acide nicotinique (ou niacine), l'alcool nicotinylique, ainsi que leurs sels et dérivés.

A titre de dérivés on peut citer les esters de l'acide nicotinique tels que, par exemple, le nicotinate de tocophéryle, les acides aminés nicotiniques, les esters d'acides carboxyliques et d'alcool nicotinylique, le N-oxyde d'acide nicotinique et le N-oxydeniacinamide.

La quantité de vitamine B3 de la composition utilisée selon la présente invention va de 0,5 à 10 %, de préférence de 1 à 5 % poids par rapport au poids total de ladite composition.

Avantageusement la teneur en vitamine B3 dans la composition utilisée correspond à la teneur maximale autorisée par la règlementation en vigueur.

La vitamine B5 correspond à l'acide pantothénique. Elle peut être présente dans le complément alimentaire sous la forme acide pantothénique ou sous la forme d'un sel de cet acide.

De préférence, le pantothénate de calcium est utilisé dans la composition selon l'invention.

La quantité de vitamine B5 de la composition utilisée selon la présente invention va de 0,05 à 5 %, de préférence de 0,5 à 3 % en poids par rapport au poids total de ladite composition.

Avantageusement la teneur en vitamine B5 de la composition utilisée selon l'invention correspond à la teneur maximale autorisée par la règlementation en vigueur.

La vitamine B6 peut être présente dans la composition utilisée selon la présente invention sous la forme d'un ou plusieurs composés tels que la pyridoxine, l'acide pyridoxique, les esters de pyridoxine tel que le tripalmitate de pyridoxine, les amines de pyridoxine telle que la pyridoxamine, ainsi que leurs sels et dérivés.

A titre de dérivés on peut citer les composés choisis dans le groupe formé par le pyridoxal, Le phosphate de pyridoxal.

De préférence, le chlorhydrate de pyridoxine est utilisé dans la composition utilisée selon la présente invention.

La quantité de vitamine B6 de la composition utilisée selon la présente invention va de 0,01 à 5 %, de préférence de 0,05 à 1 % en poids par rapport au poids total de ladite composition

Avantageusement la teneur en vitamine B6 dans la composition utilisée selon la présente invention correspond à la teneur maximale autorisée par la règlementation en vigueur.

La quantité de vitamine B8 de la composition utilisée selon la présente invention va de 0,001 à 0,5 %, de préférence de 0,005 à 0,1 % en poids par rapport au poids total de ladite composition.

Avantageusement la teneur en vitamine B8 dans la composition utilisée selon la présente invention correspond à la teneur maximale autorisée par la règlementation en vigueur.

Bien entendu, les sels utilisés dans la formulation de la composition utilisée selon la présente invention sont choisis pour leur innocuité. On peut notamment citer les sels de zinc, de cuivre et de sélénium sous formes chélatées avec des acides aminés.

La quantité de zinc la composition utilisée selon la présente invention va de 0,1 à 2 %, de préférence de 0,5 à 1,2 % en poids par rapport au poids total de ladite composition.

Avantageusement la teneur en zinc dans la composition correspond à la teneur maximale autorisée par la règlementation en vigueur.

La quantité de cuivre de la composition utilisée selon la présente invention va de 0,01 à 1 %, de préférence de 0,05 à 0,5 % en poids par rapport au poids total de ladite composition

Avantageusement la teneur en cuivre dans la composition correspond à la teneur maximale autorisée par la règlementation en vigueur.

La quantité de sélénium de la composition utilisée selon la présente invention va de 0,0005 à 0,05 %, de préférence de 0,001 à 0,01 % en poids par rapport au poids total de ladite composition

Avantageusement la teneur en sélénium dans la composition correspond à la teneur maximale autorisée par la règlementation en vigueur.

Avantageusement la composition utilisée selon l'invention comprend de la vitamine E.

De préférence, la vitamine E est utilisée dans la composition selon l'invention dans un rapport cystine/ vitamine E allant de 30 à 36, de manière préférée allant de 32 à 34.

De préférence, la quantité de vitamine E dans la composition utilisée selon l'invention va de 0,5 à 10 %, de préférence de 1 à 4 % poids par rapport au poids total de ladite composition.

Avantageusement la composition utilisée selon l'invention comprend de l'extrait de prêle.

De préférence, la quantité d'extrait de prêle dans la composition utilisée selon l'invention va de 0,1 à 2 %, de préférence de 0,5 à 1,5 % poids par rapport au poids total de ladite composition.

Avantageusement, la composition utilisée selon l'invention ne comprend pas d'extrait de groseilles à maquereau.

Avantageusement la composition utilisée selon l'invention comprend un extrait de jus de melon.

De préférence, la quantité en extrait de jus de melon dans la composition utilisée selon l'invention va de 0,5 à 2,5 %, de préférence de 1 à 2 % en poids par rapport au poids total de ladite composition.

Avantageusement, la composition utilisée selon l'invention ne comprend pas d'extrait de mandarine.

Selon un mode particulier de l'invention, la composition utilisée selon l'invention ne comprend pas d'extraits de plantes inhibiteurs de la tyrosinase.

Selon un autre mode particulier de l'invention, la composition utilisée selon l'invention ne comprend pas au moins un extrait de plantes choisi parmi les extraits de plantes suivants : extraits d'arbousier, busserole, airelle rouge, callune vulgaire, ericacées, rosacées, saxifragacées, réglisse, mûrier du Japon, artichaut, achillée millefeuille, matricaire, scutellaire, raisin, canneberge, pin maritime, pépins de raisin, angélique, polypode fougère, thé, canne à sucre, pomme, sorbier, raisin, agrumes, châtaigne, faux poivier, grenade, fraise, géranium, stellaire intermédiaire, argousier, chèvrefeuille, cassier, gaillarde aristée, onagre, origan, thym, Ginseng, Kudzu, maïs, Aloe, mûre, myrtille, chicorée, ni d'extraits de champignons, ni d'extraits d'algue brune « Arc en ciel ».

Avantageusement la composition est préparée en mélangeant les différents composants à l'état de poudres.

Avantageusement, la composition utilisée conformément à l'invention peut comprendre en outre un véhicule physiologiquement acceptable, conforme à une utilisation par voie orale, tel que notamment une phase aqueuse.

La composition utilisée conformément à l'invention peut être formulée avec les excipients usuellement utilisés dans les compositions destinées à la voie orale, notamment des agents humectants, des épaississants, des agents de textures, des agents de saveur, des agents d'enrobage, des conservateurs, des antioxydants, des colorants, des extraits de plantes

Bien entendu, l'homme du métier veillera à choisir ces excipients de manière à ne pas altérer les propriétés de ladite composition.

La composition utilisée conformément à l'invention peut être formulée selon une des présentations suivantes : une gélule, une dragée, un comprimé, une capsule molle ou dure, ou encore une boisson, une suspension, une solution, un gel.

La formulation de ladite composition conformément à l'invention met en oeuvre des procédés classiques qui font partie des compétences générale de l'homme du métier.

La composition selon la présente invention peut être obtenue en mélangeant les différents composants de la composition dans les rapports et/ ou quantités requis.

La présente invention vise encore un procédé de préparation de la composition utilisée selon l'invention comprenant les étapes suivantes :

disposer de cystine ;

disposer de glutathion ;

disposer de glycine ;

disposer de glutamine ;

disposer d'ascorbate de calcium ;

éventuellement disposer d'au moins un composant choisi dans le groupe formé par le zinc ou l'un de ses sels, le cuivre ou l'un de ses sels, le sélénium ou l'un de ses sels, les vitamines B, notamment B3, B5, B6 et/ou B8, la vitamine E, les extraits de jus de melon et les extraits de prêle, et les mélanges de ces composants;

effectuer le mélange de la cystine, du glutathion, de la glycine, de la glutamine, de l'ascorbate de calcium et du au moins un éventuel autre composant.

Comme déjà mentionné, la présente invention vise l'utilisation cosmétique, par voie orale, d'une composition à titre d'actif pour blanchir et/ou éclaircir la peau et/ou dépigmenter la peau et en particulier réduire la taille des taches brunes pigmentaires, ladite composition comprenant au moins de la cystine et du glutathion dans un rapport cystine/glutathion allant de 1,5 à 4.

L'utilisation de la composition selon l'invention n'appartient pas au domaine thérapeutique. Il s'agit d'une composition nutraceutique, non-thérapeutique.

Ladite utilisation peut aussi être qualifiée de complément alimentaire dédié à blanchir et/ou éclaircir la peau et/ou dépigmenter la peau, en particulier pour réduire la taille, atténuer voire supprimer les taches brunes de pigmentation, ou encore prévenir, réduire et/ou traiter une altération du teint de la peau. Plus particulièrement, ladite utilisation permet de réduire, atténuer, supprimer les défauts de pigmentation de la peau tels que le mélasma ou masque de grossesse, les lentigos notamment les lentigos séniles.

Le procédé de traitement cosmétique selon l'invention vise encore à prévenir et/ou traiter les signes du vieillissement de la peau en particulier les taches de sénescence.

Il existe différentes méthodes pour évaluer la couleur de la peau *in vivo* notamment des méthodes d'évaluation instrumentales et visuelles. Les méthodes d'évaluation instrumentales, plus objectives sont privilégiées notamment au motif que les effets des produits éclaircissants/blanchissants/dépigmentants produisent leurs effets lentement.

Dans le domaine cosmétique, une méthode couramment utilisée pour évaluer la couleur de la peau est la mesure des valeurs de L*a*b* par spectrophotométrie.

Le système utilisé classiquement pour évaluer la couleur est le système CIELab dans lequel sont définis les paramètres suivants :

L*, la clarté qui dérive de la luminance de la surface, définit l'axe allant du noir/foncé (0) au blanc/clair (100),

a* définit l'axe allant du vert (-100) vers le rouge (+100),

b* définit l'axe allant du bleu (-100) vers le jaune (+100).

Le paramètre L* est un bon marqueur pour évaluer l'éclaircissement/blanchiment de la peau : plus la peau est claire, plus la valeur L* est élevée.

Généralement, une variation du paramètre L* de l'ordre de 1% est un résultat significatif. Néanmoins la meilleure description de l'éclaircissement/blanchiment est donné en combinant les paramètres L* et b* pour définir l'angle de typologie individuel ou ITA° (Individuel Typology Angle) « Petit L., Pierard GE, Skin lightening products revisited, International Journal of Cosmetic Science, 2003 ; 25. 169-181. »

L'ITA° est calculé au moyen de la formule :

$$ITA°= ArcTan\ [(L\text{*-}50)/b\text{*}]\ x\ (180\ /π)$$

Une variation de l'ITA° de l'ordre de 5% est un résultat significatif.

L'évaluation des taches brunes pigmentaires notamment l'évolution de leur surface (en mm$^2$) est mesurée par pixellisation, par exemple au niveau du visage dans la zone de la joue, en comparant des photographies numériques à polarisation croisée prises en début d'expérience (jour 0),à mi-expérience (jour 45 ) et à la fin de l'expérience (jour 90).

La présente invention vise encore un procédé non-thérapeutique, cosmétique de blanchiment et/ou d'éclaircissement et/ou de dépigmentation de la peau, en particulier pour réduire la taille des taches brunes pigmentaires, comprenant une étape d'administration à un individu par voie orale d'une composition comprenant au moins de la cystine et du glutathion en un rapport cystine/glutathion allant de 1,5 à 4, de préférence 1,5 à 3, et de manière préférée de 1,5 à 2,5 et avantageusement de 1,8 à 2,2.

En particulier l'invention vise un procédé selon l'invention dans lequel on administre les doses journalières suivantes en matière active :

de la cystine en une teneur allant de 0,001 à 2 g, de préférence de 0,1 à 1,0 g et de manière préférée environ 0,3 à 0,8 g ;

du glutathion en une teneur allant de 0,001 à 1 g, de préférence de 0,05 à 0,5 g et de manière préférée environ 0,1 à 0,4 g ;

de la glycine en une teneur allant de 0,001 à 1 g, de préférence de 0,005 à 0,5 g et de manière préférée environ 0,01 à 0,1 g ;

de la glutamine en une teneur allant de 0,001 à 1 g, de préférence de 0,01 à 0,5 g et de manière préférée environ 0,05 à 0,2 g,

de l'ascorbate de calcium en une teneur allant de 0,001 à 1 g, de préférence de 0,01 à 0,5 g et de manière préférée environ 0,05 à 0,2 g.

De manière avantageuse, les doses journalières suivantes sont administrées :

de la vitamine B3 en une teneur correspondant à une teneur allant de 0,01 à 0,03 g en nicotinamide ;

de la vitamine B5 en une teneur correspondant à une teneur allant de 0,010 à 0,015 g en pantothénate de calcium ;

de la vitamine B6 en une teneur correspondant à une teneur allant de 0,001 à 0,005 g en chlorhydrate de pyridoxine ;

de la vitamine B8 en une teneur correspondant à une teneur allant de 0,0001 à 0,0005 g en biotine ;

du zinc ou l'un de ses sels en une teneur en zinc allant de 0,005 à 0,015 g ;

du cuivre ou l'un de ses sels en une teneur en cuivre allant de 0,001 à 0,002 g,

de la vitamine E en une teneur correspondant à une teneur allant de 0,005 à 0,1 g ;

de l'extrait de jus de melon concentré en une teneur allant de 0,005 à 0,015 g

de l'extrait de prêle en une teneur allant de 0,001 à 0,01 g

Le procédé selon l'invention est généralement mis en oeuvre par une administration journalière de la composition utilisée selon la présente invention ou des doses journalières selon la présente invention. Cette administration peut être effectuée en une seule prise journalière, en deux prises journalières, en trois prises journalières voire quatre prises journalières, par exemple aux heures des repas, avantageusement elle est effectuée en deux fois l'une le matin et l'autre le soir.

Le procédé selon l'invention est généralement mis en oeuvre sur une période variant d'une à plusieurs semaines voire plusieurs mois. Cette période de traitement pouvant être répétée plusieurs fois au cours d'une année.

Les exemples qui suivent visent à illustrer l'invention sans en limiter la portée.

Exemples

I/ Compositions

Une composition A selon l'invention et une composition comparative B, toutes les deux sous forme de gélules, sont préparées.

Dans le tableau 1, sont respectivement présentés les ingrédients de la composition A en mg, le pourcentage pondéral des ingrédients dans la composition A, les ingrédients de la composition B en mg.

Pour préparer les compositions A et B, les ingrédients sont pesés et mélangés à température ambiante. Les compositions A et B ainsi préparées sont stables, elles sont mises en gélules.

[Tableau 1]

| Ingrédients | Composition A selon l'invention en mg | % pds dans la composition A | Composition comparative B en mg |
|---|---|---|---|
| L-Cystine | 250,00 | 40,19 | 250,00 |
| L-Glutathion | 125,00 | 20,10 | - |
| L-Glutamine | 60,00 | 9,65 | 60,00 |
| Glycine | 30,00 | 4,82 | 30,00 |
| Vitamine E m.a. 50 % | 15,00 | 2,41 | 15,00 |
| Ascorbate de calcium | 55,00 | 8,84 | 55,00 |
| Jus de melon concentré | 10,00 | 1,61 | 10,00 |
| Extrait sec de parties aériennes de prêle | 5,00 | 0,80 | 5,00 |
| Chélate Cu-acide aminé m.a. 10% | 7,49 | 1,20 | 7,49 |
| Chélate Zn-acide aminé m.a. 20% | 24,99 | 4,02 | 24,99 |
| Chélate Se-acide aminé m.a. 1% | 3,50 | 0,56 | 3,50 |
| Vitamine B3 | 8,91 | 1,43 | 8,91 |
| Vitamine B5 (Pantothénate de Calcium) | 6,00 | 0,97 | 6,00 |
| Vitamine B6 (chlorhydrate de pyridoxine) | 1,05 | 0,17 | 1,05 |
| Vitamine B8 (biotine) | 0,15 | 0,02 | 0,15 |
| Stéarate de magnésium | 19,91 | 3,20 | 13,92 |
| Maltodextrine | - | - | 130,99 |
| Capsule | 118,00 | 0 | 118 |
| Total ingrédients + capsule | 740 | 100 | 740 |

Deux autres compositions ont été préparées : le placebo C et la composition comparative D présentées ci-dessous

Placebo C

La composition placebo C comprenait 622 mg de stéarate de magnésium dans une gélule de 118 mg.

Composition D

La composition D comprenant les ingrédients suivants présentés dans le tableau 2 est préparée par mélange des ingrédients à température ambiante Les quantités des ingrédients sont indiquées en mg.

[Tableau 2]

| Ingrédients | Composition comparative D en mg |
|---|---|
| L-Cystéine | 100 |
| L-Glutathion | 250 |
| L-Glutamine | 100 |
| Glycine | 100 |
| Vitamine E | 15 |
| Vitamine B2 | 1,7 |
| Vitamine B6 | 2 |
| Chélate Se-acide aminé m.a. 0,2% | 15 |
| Poudre de jus de melon | 100 |
| Extrait d'acerola | 50 |
| Extrait de cynorrhodon | 50 |
| Extrait d'écorce de pin | 60 |
| Lycopène m.a. 10% | 50 |
| Collagène de poisson | 100 |
| Acide alpha-lipoïque | 30 |
| Excipient | 326,3 |
| Total | 1350 |

II/ Mesures de l'activité des compositions

Les quantités journalières présentées dans le tableau 3 ont été administrées à chaque sujet pendant 90 jours.

[Tableau 3]

| | L-Cystine en mg | L-Glutathion en mg | L-Cystéine en mg |
|---|---|---|---|
| Composition A selon l'invention (2 gélules par jour) | 500 | 250 | - |
| Composition C Placebo (2 gélules par jour) | - | - | - |
| Composition comparative B (2 gélules par jour) | 500 | | |
| Composition comparative D (1 gélule par jour) | - | 250 | 100 |

L'étude a été réalisée sur 120 sujets, en double aveugle, avec répartition aléatoire : 30 sujets ont reçu la composition A, 30 sujets ont reçu la composition B, 30 sujets ont reçu la composition C et 30 sujets ont reçu la composition D.

Les compositions ont été administrées pendant 90 jours.

Sujets

Les sujets retenus répondaient aux critères suivants :

- sujets en bonne santé,

- d'âge entre 30 et 50 ans,

- peau claire correspondant à des phototypes III et IV sur l'échelle de Fitzpatrick et présentant une ou plusieurs taches brunes sur le visage c'est-à-dire au moins une tache dont la plus petite dimension était d'au moins 2.5 mm de diamètre.

La couleur de la peau a été évaluée par spectrophotométrie au moyen d'un spectrophotomètre/colorimètre CM 700D (Konica-Minolta) par mesure des valeurs de L\*a\*b\* et calcul de la valeur de l'angle de typologie individuel ou ITA° (Individual Typology Angle).

Les paramètres L\* de d'ITA° permettent de mesurer le blanchiment/l'éclaircissement de la peau.

L'évaluation de la réduction des taches brunes a été effectuée en mesurant la taille des taches sur des photographies numériques à polarisation croisée.

Les mesures ont été effectuées à J=0, à J 45 (après 45 jours de traitement) et à J 90 (après 90 jours de traitement).

Le tableau 4 présente l'évolution de chaque critère (en %) par rapport au début de l'étude à J0 et pour chaque composition.

[Tableau 4]

| | Composition A (2 gélules par jour) | Placebo C (2 gélules par jour) | Composition comp. B (2 gélules par jour) | Composition comp. D (1 gélule par jour) |
|---|---|---|---|---|
| Taille des taches brunes[1] à J 45 | **-16,2%\*^** | +4,3% | +1,4% | -4,4% |
| Taille des taches brunes[1] à J 90 | **-34,4%\*^** | +4,4% | +4,8% | -3,6% |
| ITA° mesuré sur la joue[2] à J 45 | **+2,2%** | -3,5% | -1,8% | -1,0% |
| ITA° mesuré sur la joue[2] à J 90 | **+8,7%\*^** | +0,5% | -0,8% | -1,0% |
| L\*mesuré sur la joue[3] à J 45 | **+0,6%** | -0,1% | -0,1% | +0,3% |
| L\* mesuré sur la joue[3] à J 90 | **+1,8%\*^** | +0,4% | -0,1% | +0,1% |
| ITA° mesuré sur le poignet[2] à J 45 | **+5,1%\*** | -2,6% | -2,0% | -1,1% |
| ITA° mesuré sur le poignet (2) à J 90 | **+6,7%\*** | -0,7% | +3,9% | +2,0% |
| L\*mesuré sur le poignet[3] à J 45 | **+0,8%** | -0,4% | -0,5% | -0,3% |
| L\* mesuré sur le poignet[3] à J 90 | **+1,1%\*** | -0,1% | +0,4% | 0,0% |
| [1] : mesure de l'évolution en% de la surface ($mm^2$) des taches brunes, [2] : mesure de l'évolution de l'angle de typologie individuel ITA°, [3] : mesure de l'évolution de la clarté L\*, \* : évolution statistiquement significative par rapport à J0, ^ : les variations observées avec la composition A sont significativement différentes de celles observées avec les 3 autres compositions. | | | | |

La composition selon l'invention permet une réduction significative de la taille des taches brunes (- 16,2% à 45 jours et -34,4% à 90 jours), une partie des taches n'est plus visible, la peau parait plus homogène.

Elle permet également une augmentation de l'éclaircissement/du blanchiment de la peau mesuré par une augmentation du paramètre L\* à la fois sur la joue (+0,6% à 45 jours et +1,8% à 90 jours), et le poignet (+0,8% à 45 jours et +1,1% à 90 jours), et une augmentation significative de l'ITA° à la fois sur la joue (+2,2% à 45 jours et +8,7% à 90 jours) et le poignet (+5,1% à 45 jours et +6,7% à 90 jours).

La proportion de sujets présentant une réduction de la surface des taches brunes à 90 jours est de 87 % avec la composition A selon l'invention, cette valeur est élevée et significativement différente de celles obtenues avec la composition placebo C (40%), avec la composition B (50%) et avec la composition D (39%).

De même la proportion de sujets présentant un éclaircissement/blanchiment de la peau à 90 jours avec une augmentation de la valeur ITA° au niveau de la joue est de 77%, elle est de significativement élevée en comparaison à celles de la composition placebo C (43%), B (47%) et D (39%).

On observe aussi un effet blanchissant/éclaircissant sur les taches brunes

**Revendications**

1. Utilisation non-thérapeutique, cosmétique, par voie orale, d'une composition à titre d'actif pour blanchir et/ou éclaircir la peau et/ou dépigmenter la peau **caractérisée en ce que** ladite composition comprend au moins de la cystine et du glutathion dans un rapport cystine/glutathion allant de 1,5 à 4, de préférence 1,5 à 3, et de manière préférée de 1,5 à 2,5 et avantageusement de 1,8 à 2,2.

2. Utilisation selon la revendication 1 dans laquelle la quantité de cystine dans ladite composition va de 30 à 60%, de préférence de 35 à 45 % poids par rapport au poids total de ladite composition.

3. Utilisation selon la revendication 1 ou 2 dans laquelle la cystine comprend moins de 5 ppm de métaux lourds.

4. Utilisation selon l'une quelconque des revendications précédentes dans laquelle la quantité de glutathion va de 10 à 30 %, de préférence de 15 à 25 % poids par rapport au poids total de ladite composition.

5. Utilisation selon l'une quelconque des revendications précédentes dans laquelle la composition comprend de la glycine, de préférence dans un rapport cystine/glycine allant de 7 à 9,5, de manière préférée allant de 8,0 à 8,5.

6. Utilisation selon l'une quelconque des revendications précédentes dans laquelle la composition comprend de la glutamine de préférence dans un rapport cystine/glutamine allant de 3,5 à 5,0, de manière préférée allant de 4,0 à 4,5.

7. Utilisation selon l'une quelconque des revendications précédentes dans laquelle la composition comprend l'ascorbate de calcium de préférence dans un rapport cystine/ ascorbate de calcium allant de 3,5 à 5,5, de manière préférée allant de 4,0 à 5,0.

8. Utilisation selon l'une quelconque des revendications précédentes dans laquelle la composition comprend au moins un composant choisi dans le groupe formé par le zinc ou l'un de ses sels, le cuivre ou l'un de ses sels, le sélénium ou l'un de ses sels, les vitamines B, notamment B3, B5, B6 et/ou B8, la vitamine E, les extraits de jus de melon, les extraits de prêle et les mélanges de ces composants.

9. Utilisation selon l'une quelconque des revendications précédentes pour réduire la taille des taches brunes.

10. Utilisation selon l'une quelconque des revendications précédentes dans laquelle la composition ne comprend pas d'extrait de groseilles à maquereau.

11. Procédé de préparation de la composition utilisée selon l'une quelconque des revendications précédentes comprenant les étapes suivantes :

disposer de cystine ;
disposer de glutathion ;
disposer de glycine ;
disposer de glutamine ;
disposer d'ascorbate de calcium ;
éventuellement disposer d'au moins un composant choisi dans le groupe formé par le zinc ou l'un de ses sels, le cuivre ou l'un de ses sels, le sélénium ou l'un de ses sels, les vitamines B, notamment B3, B5, B6 et/ou B8, la vitamine E, les extraits de jus de melon, les extraits de prêle et les mélanges de ces composants ;
effectuer le mélange de la cystine, du glutathion, de la glycine, de la glutamine, de l'ascorbate de calcium et du au moins un éventuel autre composant.

12. Procédé non-thérapeutique, cosmétique de blanchiment et/ou d'éclaircissement et ou de dépigmentation de la peau comprenant une étape d'administration à un individu par voie orale d'une composition comprenant au moins de la cystine et du glutathion en un rapport cystine/glutathion allant de 1,5 à 4, de préférence 1,5 à 3, et de manière préférée de 1,5 à 2,5 et avantageusement de 1,8 à 2,2.

13. Procédé selon la revendication précédente dans lequel on administre les doses journalières suivantes en matière active :

de la cystine en une teneur allant de 0,001 à 2 g, de préférence de 0,1 à 1,0 g et de manière préférée environ 0,3 à 0,8 g ;
du glutathion en une teneur allant de 0,001 à 1 g, de préférence de 0,05 à 0,5 g et de manière préférée environ 0,1 à 0,4 g ;
de la glycine en une teneur allant de 0,001 à 1 g, de préférence de 0,005 à 0,5 g et de manière préférée environ 0,01 à 0,1 g ;
de la glutamine en une teneur allant de 0,001 à 1 g, de préférence de 0,01 à 0,5 g et de manière préférée environ 0,05 à 0,2 g,
de l'ascorbate de calcium en une teneur allant de 0,001 à 1 g, de préférence de 0,01 à 0,5 g et de manière préférée environ 0,05 à 0,2 g.

14. Procédé selon la revendication précédente dans lequel on administre les doses journalières suivantes :

de la vitamine B3 en une teneur correspondant à une teneur allant de 0,01 à 0,03 g en nicotinamide ;
de la vitamine B5 en une teneur correspondant à une teneur allant de 0,010 à 0,015 g en pantothénate de calcium ;
de la vitamine B6 en une teneur correspondant à une teneur allant de 0,001 à 0,005 g en chlorhydrate de pyridoxine ;
de la vitamine B8 en une teneur correspondant à une teneur allant de 0,0001 à 0,0005 g en biotine ;
du zinc ou l'un de ses sels en une teneur en zinc allant de 0,005 à 0,015 g ;
du cuivre ou l'un de ses sels en une teneur en cuivre allant de 0,001 à 0,002 g ;
de la vitamine E en une teneur correspondant à une teneur allant de 0,005 à 0,1 g ;
de l'extrait de jus de melon en une teneur allant de 0,005 à 0,015 g ;
de l'extrait de prêle en une teneur allant de 0,001 à 0,01 g.

15. Procédé non-thérapeutique, cosmétique selon l'une quelconque des revendications 12 à 14 pour réduire la taille des taches brunes pigmentaires.

**Patentansprüche**

1. Nicht-therapeutische, kosmetische, oral verabreichte Verwendung einer Wirkstoffzusammensetzung zum Bleichen und/oder Aufhellen und/oder Depigmentieren der Haut, **dadurch gekennzeichnet, dass** die Zusammensetzung mindestens Cystin und Glutathion in einem Cystin-Glutathion-Verhältnis von 1,5 bis 4, vorzugsweise von 1,5 bis 3, bevorzugt von 1,5 bis 2,5 und vorteilhafterweise von 1,8 bis 2,2 enthält.

2. Verwendung nach Anspruch 1, wobei die Cystinmenge in dieser Zusammensetzung 30 bis 60 Gew.-%, vorzugsweise 35 bis 45 Gew.-% bezogen auf das Gesamtgewicht dieser Zusammensetzung beträgt.

3. Verwendung nach Anspruch 1 oder 2, wobei das Cystin weniger als 5 ppm Schwermetalle enthält.

4. Verwendung nach einem der vorhergehenden Ansprüche, wobei die Glutathionmenge 10 bis 30 Gew.-%, vorzugsweise 15 bis 25 Gew.-% Gewicht bezogen auf das Gesamtgewicht dieser Zusammensetzung beträgt.

5. Verwendung nach einem der vorhergehenden Ansprüche, wobei die Zusammensetzung Glycin vorzugsweise in einem Cystin/Glycin-Verhältnis von 7 bis 9,5, bevorzugt von 8,0 bis 8,5 enthält.

6. Verwendung nach einem der vorhergehenden Ansprüche, wobei die Zusammensetzung Glutamin vorzugsweise in einem Cystin/Glutamin-Verhältnis von 3,5 bis 5,0, bevorzugt von 4,0 bis 4,5 enthält.

7. Verwendung nach einem der vorhergehenden Ansprüche, wobei die Zusammensetzung Calciumascorbat vorzugsweise in einem Cystin-Calciumascorbat-Verhältnis von 3,5 bis 5,5, bevorzugt von 4,0 bis 5,0 enthält.

8. Verwendung nach einem der vorhergehenden Ansprüche, wobei die Zusammensetzung mindestens eine Komponente aus der Gruppe von Zink oder einem seiner Salze, Kupfer oder einem seiner Salze, Selen oder einem seiner Salze, Vitamin B, insbesondere B3, B5, B6 und/oder B8, Vitamin E, Melonensaftextrakte, Pflaumenextrakte und Mischungen dieser Komponenten enthält.

9. Verwendung nach einem der vorhergehenden Ansprüche, um die Größe dunkler Hautflecken zu reduzieren.

10. Verwendung nach einem der vorhergehenden Ansprüche, wobei die Zusammensetzung keinen Stachelbeerextrakt enthält.

11. Verfahren zur Herstellung der verwendeten Zusammensetzung nach einem der vorhergehenden Ansprüche, das die folgenden Schritte umfasst:

Bereitstellung von Cystin;
Bereitstellung von Glutathion;
Bereitstellung von Glycin;
Bereitstellung von Glutamin;
Bereitstellung von Calciumascorbat;
gegebenenfalls Bereitstellung von mindestens einer Komponente aus der Gruppe von Zink oder einem seiner Salze, Kupfer oder einem seiner Salze,
Selen oder einem seiner Salze, Vitamin B, insbesondere B3, B5, B6 und/oder B8, Vitamin E, Melonensaftextrakte, Pflaumenextrakte und Mischungen dieser Komponenten;
Vermischung des Cystins, Glutathions, Glycins, Glutamins, Calciumascorbats und ggf. mindestens einer weiteren Komponente.

12. Nicht-therapeutisches, kosmetisches Verfahren zum Bleichen und/oder Aufhellen und/oder Depigmentieren der Haut, das die orale Verabreichung einer Zusammensetzung an ein Individuum umfasst, die mindestens Cystin und Glutathion in einem Cystin-Glutathion-Verhältnis von 1,5 bis 4, vorzugsweise 1,5 bis 3, bevorzugt von 1,5 bis 2,5 und vorteilhafterweise von 1,8 bis 2,2 enthält.

13. Verfahren nach dem vorhergehenden Anspruch, wobei die folgenden Tagesdosen der Wirkstoffe verabreicht werden:

Cystin mit einem Gehalt von 0,001 bis 2 g, vorzugsweise von 0,1 bis 1,0 g und bevorzugt von ca. 0,3 bis 0,8 g;
Glutathion mit einem Gehalt von 0,001 bis 1 g, vorzugsweise von 0,05 bis 0,5 g und bevorzugt von ca. 0,1 bis 0,4 g;
Glycin mit einem Gehalt von 0,001 bis 1 g, vorzugsweise von 0,005 bis 0,5 g und bevorzugt von ca. 0,01 bis 0,1 g;
Glutamin mit einem Gehalt von 0,001 bis 1 g, vorzugsweise von 0,01 bis 0,5 g und bevorzugt von ca. 0,05 bis 0,2 g;
Calciumascorbat mit einem Gehalt von 0,001 bis 1 g, vorzugsweise von 0,01 bis 0,5 g und bevorzugt von ca. 0,05 bis 0,2 g.

14. Verfahren nach dem vorhergehenden Anspruch, wobei die folgenden Tagesdosen verabreicht werden:

Vitamin B3 mit einem Gehalt von 0,01 bis 0,03 g Nicotinamid;
Vitamin B5 mit einem Gehalt von 0,010 bis 0,015 g Calciumpantothenat;
Vitamin B6 mit einem Gehalt von 0,001 bis 0,005 g Pyridoxinhydrochlorid;
Vitamin B8 mit einem Gehalt von 0,0001 bis 0,0005 g Biotin;
Zink oder eines seiner Salze mit einem Zinkgehalt von 0,005 bis 0,015 g;
Kupfer oder eines seiner Salze mit einem Kupfergehalt von 0,001 bis 0,002 g;
Vitamin E mit einem Gehalt von 0,005 bis 0,1 g;
Melonensaftextrakt mit einem Gehalt von 0,005 bis 0,015 g;
Pflaumenextrakt mit einem Gehalt von 0,001 bis 0,01 g.

15. Nicht-therapeutisches, kosmetisches Verfahren nach einem der Ansprüche 12 bis 14, um die Größe dunkler Pigmentflecken zu reduzieren. 1

**Claims**

1. A non-therapeutic, cosmetic, oral use of a composition as active ingredient for whitening and/or lightening the skin and/or depigmenting the skin, **characterized in that** the said composition comprises at least cystine and glutathione in a cystine/glutathione ratio ranging from 1.5 to 4, preferably from 1.5 to 3, and preferably from 1.5 to 2.5 and advantageously from 1.8 to 2.2.

2. A use according to claim 1 wherein the amount of cystine in said composition is from 30 to 60%, preferably from 35 to 45% by weight based on the total weight of said composition.

3. A use according to claim 1 or 2 wherein the cystine comprises less than 5 ppm of heavy metals.

4. A use according to any one of the preceding claims wherein the amount of glutathione ranges from 10 to 30%, preferably from 15 to 25% by weight based on the total weight of said composition.

5. A use according to any one of the preceding claims wherein the composition comprises glycine, preferably in a cystine/glycine ratio of from 7 to 9.5, preferably from 8.0 to 8.5.

6. A use according to any one of the preceding claims wherein the composition comprises glutamine preferably in a cystine/glutamine ratio of from 3.5 to 5.0, preferably from 4.0 to 4.5.

7. A use according to any one of the preceding claims wherein the composition comprises calcium ascorbate preferably in a cystine to calcium ascorbate ratio of from 3.5 to 5.5, preferably from 4.0 to 5.0.

8. A use according to any one of the preceding claims, wherein the composition comprises at least one component selected from the group formed by zinc or one of its salts, copper or one of its salts, selenium or one of its salts, vitamins B, in particular B3, B5, B6 and/or B8, vitamin E, melon juice extracts, horsetail extracts and mixtures of these components.

9. A use according to any one of the preceding claims for reducing the size of brown spots.

10. Use according to any one of the preceding claims wherein the composition does not comprise gooseberry extract.

11. A method of preparing the composition used according to any of the preceding claims comprising the following steps:

    have cystine;
    have glutathione;
    have glycine;
    have glutamine;
    have calcium ascorbate;
    optionally providing at least one component selected from the group consisting of zinc or one of its salts, copper or one of its salts, selenium or one of its salts, vitamins B, in particular B3, B5, B6 and/or B8, vitamin E, melon juice extracts, horsetail extracts, and mixtures of these components;
    effecting the mixture of cystine, glutathione, glycine, glutamine, calcium ascorbate and at least one other possible component.

12. Non-therapeutic, cosmetic method for whitening and/or lightening and/or depigmenting the skin, comprising a step of administering to an individual orally a composition comprising at least cystine and glutathione in a cystine/glutathione ratio ranging from 1.5 to 4, preferably from 1.5 to 3, and preferably from 1.5 to 2.5 and advantageously from 1.8 to 2.2.

13. A method according to the preceding claim in which the following daily doses of active material are administered:
    cystine in a content ranging from 0.001 to 2 g, preferably from 0.1 to 1.0 g and preferably about 0.3 to 0.8 g;
    glutathione in a content ranging from 0.001 to 1 g, preferably from 0.05 to 0.5 g and preferably about 0.1 to 0.4 g;
    glycine in a content ranging from 0.001 to 1 g, preferably from 0.005 to 0.5 g and preferably about 0.01 to 0.1 g ;

glutamine in an amount of 0.001 to 1 g, preferably 0.01 to 0.5 g and preferably about 0.05 to 0.2 g, calcium ascorbate in an amount of 0.001 to 1 g, preferably 0.01 to 0.5 g and preferably about 0.05 to 0.2 g.

14. A method according to the preceding claim in which the following daily doses are administered:

vitamin B3 in a content corresponding to a content ranging from 0.01 to 0.03 g of nicotinamide;
vitamin B5 in a content corresponding to a content ranging from 0.010 to 0.015 g of calcium pantothenate;
vitamin B6 in a content corresponding to a content ranging from 0.001 to 0.005 g of pyridoxine hydrochloride;
vitamin B8 in a content corresponding to a content ranging from 0.0001 to 0.0005 g of biotin;
zinc or a salt thereof in a zinc content of from 0.005 to 0.015 g; copper or a salt thereof in a copper content of from 0.001 to 0.002 g;
vitamin E in a content corresponding to a content of from 0.005 to 0.1 g;
melon juice extract in a content of from 0.005 to 0.015 g; horsetail extract in a content of from 0.001 to 0.01 g.

15. A non-therapeutic, cosmetic method according to any one of claims 12 to 14 for reducing the size of pigmentary brown spots.]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016117762 A1

- KR 20120025175 A

**Littérature non-brevet citée dans la description**

- *Ph. Eur. 9eme Ed,* vol. 2 (4), 13
- *Ph. Eur. 9eme Ed,* vol. 2 (2), 56

- *International Journal of Cosmetic Science,* 2003, vol. 25, 169-181